# EUROPEAN PATENT APPLICATION

(11) **EP 4 442 253 A1**
(43) Date of publication of application: **09.10.2024**
(21) Application number: 24167900.0
(22) Date of filing: 29.03.2024
(51) Int. Cl.: A61K 9/16, A61K 9/20, A61K 9/28, A61K 31/00

(54) **A FILM COATED TABLET COMPRISING EMPAGLIFLOZIN AND SURFACTANT**

(30) Priority: 04.04.2023 TR 202303670
(71) Applicant: SANOVEL ILAC SANAYI VE TICARET A.S., Istanbul 34460 (TR)
(72) Inventor: SUNEL, Fatih, Istanbul (TR); PEHLIVAN AKALIN, Nur, Istanbul (TR); ARMUT, Merve, Istanbul (TR)
(74) Representative: Genç Ilhan, Oznur

(57) **Abstract**

The present invention relates to a film coated tablet comprising empagliflozin or a pharmaceutically acceptable salt thereof or crystalline polymorph thereof wherein comprising a surfactant and the amount of surfactant is between 0.1% and 5.0% by weight in the total core tablet. The present invention also relates to a simple, rapid, cost effective, time-saving and industrially convenient method of preparing the formulation.

## Description

### Field of the Invention

The present invention relates to a film coated tablet comprising empagliflozin or a pharmaceutically acceptable salt thereof or crystalline polymorph thereof wherein comprising a surfactant and the amount of surfactant is between 0.1% and 5.0% by weight in the total core tablet. The present invention also relates to a simple, rapid, cost effective, time-saving and industrially convenient method of preparing the formulation.

### Background of the Invention

Empagliflozin is a potent inhibitor of sodium-glucose co-transporter type 2 (SGLT-2) and is therefore used to treat type 2 diabetes. It is currently approved for the treatment of type 2 diabetes and improvement of blood sugar control.

Empagliflozin is a white to yellowish non-hygroscopic crystalline solid, very slightly soluble in water (pH 1-7.4), slightly soluble in acetonitrile and ethanol, sparingly soluble in methanol, and practically insoluble in toluene. The chemical name of empagliflozin (1S)-1,5-anhydro-1-(4-chloro-3-{4-[(3S)-tetrahydrofuran-3-yloxy]benzyl}phenyl)-D- glucitol and its chemical structure is shown in the Formula- I.

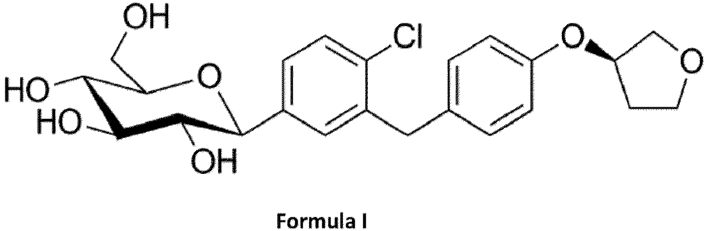

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

The first mention of the crystalline form of empagliflozin can be found in patent application WO 2006/117359. WO2006117360 discloses two other forms of empagliflozin which is described as Form I and Form II. Also, it disclosed methods for production thereof and synthesis of Empagliflozin.

US 20110014284 describes pharmaceutical compositions comprising empagliflozin, process for the preparation of such compositions and their use in the treatment of various conditions including type 1 diabetes mellitus and type 2 diabetes mellitus.

EP1730131 discloses Empagliflozin, process for production thereof, its use and pharmaceutical composition thereof. The composition of a tablet is disclosed that comprises active ingredient in admixture with pharmaceutically acceptable excipients, such as lactose, com starch, polyvinylpyrrolidone, magnesium stearate.

As evident from the prior art and the literature there are reported several pharmaceutical compositions comprising Empagliflozin. There is an existing and continual need for oral dosage formulations comprising Empagliflozin or a pharmaceutically acceptable salt thereof that have the desired dissolution profile, stability, content uniformity, improved compressibility, flowability and manufactured by safe, effective, easy manufacturing methods.

We found that the use of a certain amount of surfactant in the tablet comprising empagliflozin or a pharmaceutically acceptable salt thereof or crystalline polymorph thereof provided the desired dissolution profile and even stability.

### Detailed Description of the Invention

The main object of the present invention is to provide a film coated tablet comprising Empagliflozin or a pharmaceutically acceptable salt thereof or crystalline polymorph thereof with the desired dissolution profile.

Another object of the present invention is to provide a film coated tablet comprising Empagliflozin or a pharmaceutically acceptable salt thereof or crystalline polymorph thereof with improved compressibility, flowability, homogeneity and stability.

Another object of the present invention is to provide a film coated tablet comprising Empagliflozin or a pharmaceutically acceptable salt thereof or crystalline polymorph thereof prepared by simple, easy, time-saving and fast manufacturing methods.

Several pharmaceutical dosage forms of empagliflozin have been published. The main problem encountered when preparing formulations of empagliflozin is low solubility of empagliflozin, leading to difficulties with disintegration and dissolution times.

When a surfactant is used in a tablet comprising empagliflozin, the tablet shows the desired dissolution profile, and the surfactant also helps to provide stability. In this formulation, we seen that even if used a surfactant in small quantities, it is dissolved in water without precipitation. This also helped to provide both stability and homogeneity.

According to one embodiment of this invention, a film coated tablet comprises empagliflozin or a pharmaceutically acceptable salt thereof or crystalline polymorph thereof wherein comprising a surfactant and the amount of surfactant is between 0.1% and 5.0% by weight in the total core tablet.

Suitable surfactants are selected from the group comprising sodium lauryl sulfate, docusate sodium, glyceryl esters, glyceryl monoleate, poloxamer, polyethylene alkyl ethers, polyglyceryl esters, polysorbates, polyoxyetylene esters, polyoxyetylene stearates, sodium stearate, calcium oleate or mixtures thereof.

According to one embodiment of this invention, the surfactant is sodium lauryl sulfate. Sodium lauryl sulfate (SLS) is an alkaline, anionic surfactant. It can effectively improve the solubility of empagliflozin in water and enable it to be quickly dispersed and dissolved.

According to one embodiment of this invention, the amount of sodium lauryl sulfate is between 0.1% and 5.0% or 0.5% and 3.0% by weight in the total core tablet.

According to one embodiment of the present invention, the amount of Empagliflozin is present between 0.5% and %25.0 by weight in the total core tablet. Preferably, the amount of Empagliflozin is between 1.0% and 20.0% or 3.0% and 14.0% by weight in the total core tablet.

According to one embodiment of the present invention, preferably, empagliflozin is in the form of crystalline form.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agent and the excipients.

According to this embodiment of the present invention, the film coated tablet further comprises at least one pharmaceutically acceptable excipient is selected from the group comprising disintegrants, fillers, binders, lubricants or mixtures thereof.

Suitable disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

According to this embodiment of the present invention, the amount of disintegrants is between 1.0% and 10.0% by weight of the total core tablet. Preferably, the amount of disintegrants is between 1.0% and 4.0% by weight of the total core tablet.

According to this embodiment of the invention, the disintegrant is croscarmellose sodium.

We found that when a surfactant (especially sodium lauryl sulfate) is present with a disintegrant (especially croscarmellose sodium) in a tablet comprising empagliflozin, the desired flowability and homogeneity is provided. Also, this helps to provide the desired dissolution profile.

According to this embodiment of the present invention, the film coated tablet comprises;
- Empagliflozin
- Sodium lauryl sulfate
- Croscarmellose sodium.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals, polysorbate 80 or mixtures thereof.

According to an embodiment of the present invention, the amount of fillers are 50.0% to 95.0% by weight in the total core tablet. Preferably, it is between 70.0% to 92.0% or between 75.0% and 90.0% by weight in the total core tablet.

According to one embodiment of the present invention, the filler is microcrystalline cellulose.

According to one embodiment of the present invention, the filler is lactose monohydrate.

According to one embodiment of the present invention, the fillers are microcrystalline cellulose and lactose monohydrate.

Suitable binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 10.0% by weight of the total core tablet. Preferably, the amount of binders is between 1.0% and 4.0% by weight of the total core tablet.

According to this embodiment of the present invention, the binder is hydroxypropyl cellulose. Especially, hydroxypropyl cellulose LF is used.

Suitable lubricants are selected from the group comprising magnesium stearate, calcium stearate, zinc stearate, talc, boric acid, hydrogenated vegetable oil, sodium chlorate, magnesium lauryl sulfate, polyethylene glycol, stearic acid, fatty acid, fumaric acid or mixtures thereof.

According to this embodiment of the invention, the lubricant is magnesium stearate.

According to an embodiment of the present invention, the film coated tablet comprises;
a) Empagliflozin
b) Sodium lauryl sulfate
c) Microcrystalline Cellulose
d) Lactose monohydrate
e) HPC LF
f) Croscarmellose sodium
g) Magnesium stearate

According to one embodiment of the present invention, the film coated tablet comprising empagliflozin or a pharmaceutically acceptable salt thereof is obtained by wet granulation process. During wet granulation, solvent is used.

Suitable solvents are selected from the group comprising water, propanol, 1-butanol, 2-butanol, ethyl acetate, ethyl ether, methylene chloride, acetone, ethanol, dichloromethane or mixtures thereof.

According to one embodiment of the present invention, a process for the preparation of the film coated tablet comprises;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and then mixing,
- Adding sodium lauryl sulfate and then mixing,
- Adding magnesium stearate and then mixing,
- Compressing the mixture into the core tablet,
- Coating the core tablets with film coating agent.

### Example 1: Film coated tablet comprising empagliflozin

| **Ingredients** | **Amount (% by weight of the total core tablet)** |
|---|---|
| Empagliflozin | 0.5 - 25.0 |
| Sodium lauryl sulfate | 0.1 - 5.0 |
| Microcrystalline Cellulose PH101 | 15.0 - 30.0 |
| Lactose Monohydrate | 45.0 - 70.0 |
| HPC LF | 1.0 - 10.0 |
| Croscarmellose sodium | 1.0 - 10.0 |
| Magnesium stearate | 0.1 - 3.0 |
| **TOTAL CORE TABLET** | **100** |

### Example 2: Film coated tablet comprising empagliflozin

| **Ingredients** | **Amount (% by weight of the total core tablet)** | **Amount (% by weight of the total core tablet)** |
|---|---|---|
| Empagliflozin | 12.50 | 4.0 |
| Sodium lauryl sulfate | 2.00 | 1.00 |
| Microcrystalline Cellulose PH101 | 25.00 | 25.00 |
| Lactose Monohydrate | 55.0 | 64.5 |
| HPC LF | 3.00 | 3.00 |
| Croscarmellose sodium | 2.0 | 2.0 |
| Magnesium stearate | 0.50 | 0.50 |
| **TOTAL CORE TABLET** | **100** | **100** |

A process for example 1 or 2;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and then mixing,
- Adding sodium lauryl sulfate and then mixing,
- Adding magnesium stearate and then mixing,
- Compressing the mixture into the core tablet,
- Coating the core tablets with film coating agent.

## Claims

1. A film coated tablet comprises empagliflozin or a pharmaceutically acceptable salt thereof or crystalline polymorph thereof wherein comprising a surfactant and the amount of surfactant is between 0.1% and 5.0% by weight in the total core tablet.

2. The film coated tablet according to claim 1, wherein surfactants are selected from the group comprising sodium lauryl sulfate, docusate sodium, glyceryl esters, glyceryl monoleate, poloxamer, polyethylene alkyl ethers, polyglyceryl esters, polysorbates, polyoxyetylene esters, polyoxyetylene stearates, sodium stearate, calcium oleate or mixtures thereof.

3. The film coated tablet according to claim 1, wherein the surfactant is sodium lauryl sulfate.

4. The film coated tablet according to claim 1, wherein the amount of Empagliflozin is present between 0.5% and %25.0 by weight in the total core tablet.

5. The film coated tablet according to claim 1, wherein the film coated tablet further comprising at least one pharmaceutically acceptable excipient is selected from the group comprising disintegrants, fillers, binders, lubricants or mixtures thereof.

6. The film coated tablet according to claim 5, wherein disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

7. The film coated tablet according to claim 6, wherein the amount of disintegrants is between 1.0% and 10.0% by weight of the total core tablet.

8. The film coated tablet according to claim 6, wherein the disintegrant is croscarmellose sodium.

9. The film coated tablet according to claim 5, wherein comprising;
- Empagliflozin
- Sodium lauryl sulfate
- Croscarmellose sodium.

10. The film coated tablet according to claim 5, wherein fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals, polysorbate 80 or mixtures thereof.

11. The film coated tablet according to claim 5, wherein binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

12. The film coated tablet according to claim 11, wherein the binder is hydroxypropyl cellulose.

13. The film coated tablet according to claim 5, wherein comprising;
a) Empagliflozin
b) Sodium lauryl sulfate
c) Microcrystalline Cellulose
d) Lactose monohydrate
e) HPC LF
f) Croscarmellose sodium
g) Magnesium stearate

14. A process for the preparation of the film coated tablet comprises at following steps;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and then mixing,
- Adding sodium lauryl sulfate and then mixing,
- Adding magnesium stearate and then mixing,
- Compressing the mixture into the core tablet,
- Coating the core tablets with film coating agent.
